(19) Europäisches Patentamt
European Patent Office
Office européen des brevets

(11) Publication number : 0 552 018 A1

(12) EUROPEAN PATENT APPLICATION

(21) Application number : 93300187.7

(51) Int. Cl.$^5$ : A61K 31/445

(22) Date of filing : 13.01.93

(30) Priority : 13.01.92 US 820108

(43) Date of publication of application : 21.07.93 Bulletin 93/29

(84) Designated Contracting States : AT BE CH DE DK ES FR GB GR IE IT LI NL PT SE

(71) Applicant : Erbamont Inc.
7001 Post Road
Dublin, Ohio 43017 (US)

(72) Inventor : Banks, Phillip L.
4048 Kilbannon Way
Dublin, Ohio 43017 (US)
Inventor : Wolgemuth, Richard L.
10202 Kimberley Drive
Plain City, Ohio 43064 (US)
Inventor : Wynne, Beverley A.
1138 Strathaven Drive, N
Worthington, Ohio 43085 (US)

(74) Representative : Deans, Michael John Percy et al
Lloyd Wise, Tregear & CO. Norman House
105-109 Strand
London WC2R OAE (GB)

# (54) Use of Rifabutin for delaying or preventing mycobacterial infections.

(57) The invention relates to a method of preventing or delaying onset of a mycobacterial infection in an immunocompromised patient exposed to mycobacterial organisms which comprises administering to said patient an effective therapeutic amount of rifabutin.

EP 0 552 018 A1

This invention is concerned with delaying or preventing mycobacterial infections.

The immune systems of immunocompromised patients are so suppressed or deficient that the patient is greatly at risk of contracting a variety of mycobacterial infections as well as a wide variety of other opportunistic infections. Further, it is not uncommon for a severely immunocompromised person to suffer from more than one opportunistic infection.

Serious infections caused by *Mycobacterium avium* complex (MAC) organisms have been increasing over the last three decades. Increase in disseminated infection caused by MAC has been observed in immunocompromised patients and is concurrent with the epidemic due to acquired immunodeficiency syndrome (AIDS). Previous to the AIDS epidemic *Mycobacteriaum avium* was believed to cause disease in swine and chickens, but rarely in humans. Now, MAC infection is estimated to occur in up to 27% of patients with AIDS who have large numbers of mycobacteria in their tissues.

Prior to the AIDS epidemic the mycobacterial diseases tuberculosis and leprosy were all but eradicated in the United States. Incidence of tuberculosis and more disturbingly, tuberculosis refractive to conventional drug therapies is on the rise.

Atypical mycobacterioses infections, formerly relatively rare and benign, are today becoming more frequent and severe in immunocompromised patients, such as patients with AIDS. These infections represent a signficant problem and no approved prophylaxis or treatment is available.

Although there is no approved treatment for mycobacterial infections using rifabutin, reports in the literature indicate that rifabutin has been used to treat mycobacterial infections as, for example, tuberculosis, and *Mycobacterium avium* complex (MAC) infections in immunocompromised patients, e.g, AIDS patients. Surprisingly it has been found that administration of rifabutin to an immuno-compromised patient (especially, to an AIDS patient) at risk of getting a mycobacterial infection, will prevent or delay onset of such infections.

The invention features use of rifabutin in the manufacture of a medicament for use in preventing or delaying onset of a mycobacterial infection in an immunocompromised patient exposed to mycobacterial organisms.

The term "mycobacteria" or "mycobacterial organism" refers to a microorganism of the genus *Mycobacterium*, family Mycobacteriaceae, and of the order Actinomycetales, occuring as a gram-positive slender rod and distinguished by acid-fast staining.

Members of the genus known to have caused infections in humans include - *Mycobacterium avium* complex (MAC), *Mycobacterium kansaii, Mycobacterium marinum, Mycobacterium phlei, Mycobacterium ulcerans, Mycobacterium xenopi, Mycobacterium gordonae, Mycobacterium terrae* complex, *Mycobacterium haemophilum, Mycobacterium fortuitum, Mycobacterium tuberculosis, Mycobacterium leprae, Mycobacterium scrofulaceum and Mycobacterium smegmatis*.

Mycobacterial organisms are ubiquitous in the environment. Data indicates that MAC is acquired by ubiquitous environmental exposure; since organisms have been recovered from food, water and soil, such exposure will be difficult to prevent. [See: Horsburgh, Jr., C. Robert. *Mycobacterium Avium Complex Infection In the Acquired Immunodeficiency Syndrome,* The New England J. of Med., May 9, 1991, pp. 1332-38.]

As used herein the term "exposed" or "exposure" refers to ubiquitous environmental exposure, e.g., to food, water and soil as in the case with MAC or to exposure to a carrier or tissue or bodily fluids of a carrier for the organism as, for example, exposure to a carrier of tuberculosis or leprosy.

As used herein the term "immunocompromised patient" refers to a patient whose immune system is deficient or suppressed as indicated by reduced absolute lymphocyte count, depressed CD4(T-helper) lymphocyte count or decreased CD4/CD8(helper/suppressor) ratio which renders the immunocompromised patient susceptible to a variety of opportunistic infections including mycobacterial infections.

Immunodeficiency or immune system suppression may result from drug therapy such as high-dose or long-term systemic corticosteroid therapy or other immunosuppressive/cytotoxic therapy, diseases such as Hodgkin's disease, non-Hodgkin's lymphoma, lymphocytic leukemia, multiple myeloma, any other cancer of the lymphoreticular or histiocytic tissue, or angioimmunoblastic lymphadenopathy; genetic (congenital) immunodeficiency syndrome; chronic lung disease, e.g., bronchiectasis, emphysema, chronic bronchitis; or acquired immunodeficiency syndrome (AIDS).

As used herein the term "AIDS" is used to refer to an illness characterized by the presence of human immunodeficiency virus (HIV) infection.

In the absence of laboratory evidence of HIV infection, AIDs is diagnosed if the following indicator diseases are definitively diagnosed:

1. candidiasis of the esophagus, trachea, bronchi, or lungs;
2. cryptococcosis, extrapulmonary;
3. cryptosporidiosis with diarrhea persisting >1 month;
4. cytomegalovirus disease of an organ other than liver, spleen, or lymph nodes in a patient >1 month of age;

5. herpes simplex virus infection causing a mucocutaneous ulcer that persists longer than one month; or bronchitis, pneumonitis, or esophagitis for any duration affecting a patient >1 month of age;
6. Kaposi's sarcoma affecting a patient <60 years of age;
7. lymphoma of the brain (primary) affecting a patient <60 years of age;
8. lymphoid interstitial pneumonia and/or pulmonary lymphoid hyperplasia (LIP/PLH complex) affecting a child >13 years of age;
9. *Mycobacterium avium* complex or *M. kansaii* disease, disseminated (at a site other than or in addition to lungs , skin, or cervical or hilar lymph nodes);
10. *Pseumocystis carinii* pneumonia;
11. progressive multifocal leukoencephalopathy; or
12. toxoplasmosis of the brain affecting a patient >1 month of age.

If laboratory evidence of HIV infection is present, the presence of one or more of the diseases listed above as well as of certain other indicator diseases indicates a diagnosis of AIDS. Even if laboratory test results are negative for HIV infection, AIDS may be diagnosed if all other non-AIDS causes of immunodeficiency are ruled out and the patient has had an opportunistic infection such as mycobacterial disease or *Pneumoncystis carinii* pneumonia and the patient's T-helper/inducer (CD4) lymphocyte count is $\geqq 400/mm^3$.

In accordance with teachings of the present invention, rifabutin is administered in a suitable pharmaceutical carrier to the immunocompromised patient as a prophylactic to prevent or delay onset of mycobacterial infection.

As used herein the term "effective therapeutic amount" refers to an amount (dosage) of rifabutin sufficient to prevent or delay the onset of a mycobacterial infection in an immunocompromised patient exposed to mycobacterial organisms. Generally, an effective therapeutic amount will be in the range of from about 150 mg to about 900 mg per day, preferably from about 200 mg to about 500 mg/day and more preferably at about 300 mg/day.

It is preferred that rifabutin be orally administered to a patient in the methods of the invention. However, administration of an "effective therapeutic amount" of rifabutin by other than the oral route, for example, by intravenous injection is contemplated for use in the methods of the invention.

Generally, in the clinical studies described in Example 1, rifabutin was administered once a day. However, it is within the skill and discretion of the physician to choose a dosing schedule to meet the needs of an individual patient; for example, depending on such factors as the overall health of the patient, tolerability of the dose, number of other drugs which are being concomitantly administered to the patient, and the like, the physician might recommend a dosing schedule of every-other-day, two times a week, half of the dose in the morning, half in the evening, and the like.

Rifabutin is the generic name of the chemical compound 4-dioxo-3,4-[2-spiro-(N-isobutyl-4-piperidyl)-2,5-dihydro-1H-imidazo]-rifamycin S. Depending on the system used to chemically name a chemical compound may also be identified as 6,9-dihydro-5,17,19,21-tetrahydroxy-8,9-[2-spiro-(N-isobutyl-4-piperidyl)-2,5-dihydro-1*H*-imidazo]-23-methoxy-2,4,12,16,18,20,22-heptamethyl-6-oxo-2,7-(epoxypentadeca-[1,11,13]-trienimino)napthol[2,1-*b*]furan-1,11-(2*H*)-dione-21-acetate; or,

(9*s*)12*E*,14*S*,15*R*,16*S*,17*R*,18*R*,19*R*,20*S*,21*S*,21*S*,22*E*,24*Z*)-6,16,18,20-tetrahydroxy-1'-isobutyl-14-methyoxy-7,9,15,17,19,21,25-heptamethyl-spiro[9,4-(epoxypentadeca[1,11,13]trienimino-2*H*-furo[2',3':7,8]napth[1,2-*d*]imidazole-2,4'-piperidine-5,10,26-(3*H*,9*H*)-trione-16-acetate.

Rifabutin has the structure formula -

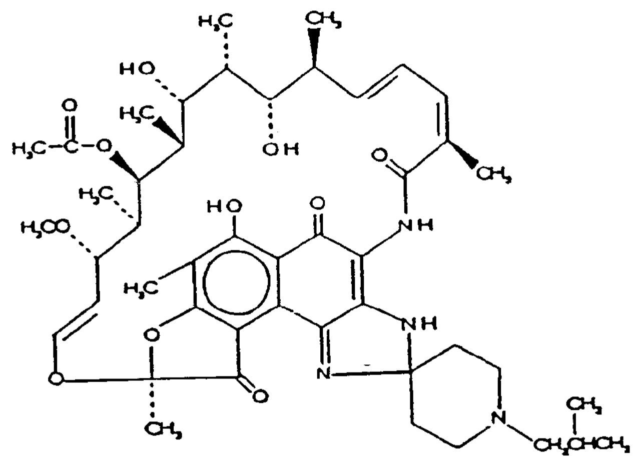

Molecular Formula: $C_{46}H_{62}N_4O_{11}$
Molecular Weight : 847.02

The preparation of rifabutin is described in U.S. Patent 4,219,478, issued August 26, 1980. Rifabutin is a red-violet powder soluble in chloroform and methanol, and very slightly soluble in water; it has a melting point of 148°C-156° (with decomposition).

In the human clinical trials which are described in Example 1, rifabutin was administered in an oral capsule containing 150 mg rifabutin per capsule. The capsules also contained as inactive ingredients, microcrystalline cellulose, magnesium stearate, red iron oxide, silica gel, sodium lauryl sulfate, titanium oxide, and (color) edible ink.

The following non-limiting example is illustrative of the practice of the method of the invention. Example 1 describes the results of a large multicenter, prospectively randomized placebo controlled clinical trial which was conducted in the United States.

Example 1

Results of Study Using Rifabutin to Prevent or Delay Mac Infection in AIDS Patients

The efficacy of rifabutin on the prevention of MAC disease in HIV positive patients having a CD4 count of ≦200/mm³ was assessed in a large multicenter controlled trial in the United States.

Five hundred ninety (590) patients with CD4 counts ≦200/mm³; and with no clinical evidence of MAC infection (as determined by two blood cultures) were randomly assigned to receive either rifabutin monotherapy (300 mg/day) or placebo. Nine of these patients, five in the rifabutin group and four in the placebo group, who were enrolled with presumptive negative evidence of MAC blood cultures were subsequently found to have had MAC bacteremia at baseline and were excluded from the study. Fifty-six of the 581 patients who had confirmed negative cultures at baseline developed MAC infection after having enrolled in the study. Eighteen of those 56 patients were in the rifabutin group and the other 38 were in the placebo group. After excluding seven patients from each group who were considered to be inevaluable, the treatment groups were compared with respect to the incidence of MAC infection; the observed difference was highly statistically significant (p=0.002, Chi-squared test). The results are reported in Table 1.

**TABLE 1**

**INCIDENCE OF MAC INFECTION**

| Number of: | TREATMENT GROUP | |
|---|---|---|
| | RIFABUTIN | PLACEBO |
| Patients with MAC | 11 | 31 |
| Patients without MAC | 269 | 256 |
| Totals | 280 | 287 |

$$x^2 = 9.76$$

$$P \cong 0.002$$

The data in Table 1 illustrates that the administration of rifabutin to AIDS patients (by definition immunocompromised patients) who are susceptible to MAC infection significantly prevents occurrence of the disease. Moreover, analysis of the data by Kaplan/Meier Methodology [See: Kalbfleisch, JD, Prentice RL (auths); *The Statistical Analysis of Failure Time Data.* New York NY: John Wiley & Sons Inc, 1980]. indicates that those patients who received rifabutin who got a MAC infection, got the MAC infection significantly later than patients on placebo. Stated another way, the data indicates that, at any point in time, patients on the placebo arm were, on average; 2.3 times more likely to die or to experience a MAC event (i.e., become infected) than those patients receiving rifabutin.

Three patients on the placebo arm of the study developed *M. tuberculosis* infections (other than MAC) while no patients receiving rifabutin developed tuberculosis. One patient on the placebo arm developed atypical mycobacterial infection while no patients receiving rifabutin developed atypical mycobacterial infection.

Patients entered into the placebo controlled multicenter Study described in Example 1 were AIDS patients who had CD4 counts of $\leqq 200/mm^3$. In order to be entered into the Study the patient had to meet the following criteria:

i) Age, 18 years or older.
ii) Positive serology test for HIV by Elisa confirmed by another method (e.g., Western Blot).
iii) Male or non-pregnant females. Women of child-bearing potential must utilize contraception.
iv) CD4 count of $\leqq 200/mm^3$ and a diagnosis of AIDS.
v) Serum liver function tests as follows: SGOT/AST < five times the upper limit of normal and alkaline phosphatase < three times the upper limit of normal and total bilirubin $\leqq 2.5$ mg.
vi) Serum creatinine $\leqq$*1.5*2.0 mg percent.
vii) Hematologic tests as follows: absolute neutrophili count $\geqq 1{,}000/mm^3$, hemoglobin $\geqq 9.5$ gm/dl, hematocrit $\geqq 29$ percent, platelet count$\geqq 75{,}000/mm^3$.
viii) No evidence of disseminated MAC disease as evaluated by two negative blood cultures and two negative stool cultures, one taken within 14 days of pretreatment.
ix) Negative tuberculin skin test as defined by a PPD $\leqq$5mm.
x) Written informed consent obtained.

Patients excluded from entry into the studies were those with known hypersensitivity to rifabutin, rifampin or other rifamycins; previous (or current) infection due to MAC or other mycobacterial disease, e.g., tuberculosis, those who were pregnant or nursing mothers and patients who had taken antiretroviral drugs (e.g., ZDV or ddl) or antimycobacterial drugs (e.g., rifampin, clofazimini, ciprofloxacin, etc.) within the last four weeks.

Rifabutin was supplied as an opaque orange capsule containing 150 mg of rifabutin. Two capsules, each containing 150 mg rifabutin, were administered as a single dose of two, 150 mg capsules with breakfast, or once a day with the morning meal and if not tolerated, two divided doses of 150 mg each with morning and evening meals.

Concomitant therapy with antimycobacterial drugs was not allowed.

Prior to being placed on study, patients were evaluated; a medical history was taken, physical given and clinical signs and symptoms assessed for, among other signs, fevers, diarrhea, night sweats, abdominal pain, fatigue, CD4 count, hematology, serum chemistries and blood culture for mycobacteria and pregnancy test given, if applicable.

During treatment the following procedures or evaluations were performed at each visit: medical history and physical exam; Karnofsky ambulatory status performance; CBC; platelet count and ANC; serum chemistries; CD4 count and blood and stool cultures for mycobacteria.

## Claims

1. Use of rifabutin in the manufacture of a medicament for use in preventing or delaying onset of a mycobacterial infection in an immunocompromised patient exposed to mycobacterial organisms.

2. Use of rifabutin as specified in Claim 1, wherein said immunocompromised patient is an AIDS patient.

3. Use of rifabutin as specified in Claims 1 or 2, wherein said mycobacterial organism is *Mycobacterium avium* complex (MAC) , *Mycobacterium kansaii, Mycobacterium marinum, Mycobacterium phlei, Mycobacterium ulcerans, Mycobacterium xenopi, Mycobacterium gordonae, Mycobacterium terrae* complex, *Mycobacterium haemophilum, Mycobacterium fortuitum, Mycobacterium tuberculosis, Mycobacterium leprae, Mycobacterium scrofulaceum* or *Mycobacterium smegmatis.*

European Patent Office

# EUROPEAN SEARCH REPORT

Application Number
EP 93 30 0187
Page 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | ANTIMICROB.AGENTS CHEMOTHER. vol. 34, no. 9, 1990, pages 1629 - 32 'Effect of Rifabutin on Disseminated Mycobacterium avium Infections in Thymectomized, CD4 T-Cell-Deficient Mice' * Abstract * * page 1629, left column, line 12 - line 15 * * page 1630, right column, line 20 - page 1631, left column, line 12 * * page 1362 * --- | 1-3 | A61K31/445 |
| X | AM.REV.RESP.DIS. vol. 139, no. 4(2), 1989, page A146 'Ansamycin (LM 427, Rifabutin) in combination with Clofazimine, Isoniazid and Ethambutol fro the Treatment of Mycobacterial Infections in AIDS Patients' * third abstract * --- | 1-3 | |
| X | NED.TIJDSCHR.GENEESK. vol. 135, no. 52, 1991, pages 2485 - 9 'Mycobacterium avium-ziekte bij AIDS-patienten; diagnostiek en therapie' * page 2486, right column, line 33 - page 2487, left column, line 19 * * page 2487; table 3 * * page 2488, left column, line 51 - line 58 * --- -/-- | 1-3 | TECHNICAL FIELDS SEARCHED (Int. Cl.5 ) A61K |

The present search report has been drawn up for all claims

| Place of search | Date of completion of the search | Examiner |
|---|---|---|
| THE HAGUE | 29 MARCH 1993 | GERLI P.F.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent Office

# EUROPEAN SEARCH REPORT

Application Number
EP 93 30 0187
Page 2

| Category | Citation of document with indication, where appropriate, of relevant passages | Relevant to claim | CLASSIFICATION OF THE APPLICATION (Int. Cl.5 ) |
|---|---|---|---|
| X | J.CHEMOTHER. vol. 3, no. 6, 1991, pages 357 - 62 'In vitro activity of Clarithromycin Alone or in Combination with Other Antimycrobial Agents against Mycobacterium avium-intracellulare. Complex StrainsIsolated from AIDS Patients' * page 359, left column, line 18 - right column, line 16 * * page 361, right column, line 18 - line 25 * * page 361, right column, line 50 - page 362, left column, line 21 * | 1-3 | |
| X | PATHOL.BIOL. vol. 39, no. 5, 1991, pages 429 - 35 'Action des antibiotiques sur des variantes pigmentés ou non pigmentés de Mycobacterium avium-intracellulare' * page 432; tables I,II * * page 434; figures 1,2 * | 1-3 | TECHNICAL FIELDS SEARCHED (Int. Cl.5 ) |

The present search report has been drawn up for all claims

| Place of search | Date of completion of the search | Examiner |
|---|---|---|
| THE HAGUE | 29 MARCH 1993 | GERLI P.F.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)